# EUROPÄISCHE PATENTSCHRIFT

(11) **EP 4 017 696 B1**
(45) Veröffentlichungstag und Bekanntmachung des Hinweises auf die Patenterteilung: **22.01.2025**
(21) Anmeldenummer: 20761166.6
(22) Anmeldetag: 18.08.2020
(51) Int. Cl.: B29C 33/40, B29C 43/02, B29C 33/42, A61M 37/00, B29L 31/00, B29C 59/02, B29K 67/00

(54) **VORRICHTUNG UND VERFAHREN ZUR HERSTELLUNG VON MIKROSTRUKTUREN**
DEVICE AND METHOD FOR MANUFACTURING MICROSTRUCTURES
DISPOSITIF ET PROCÉDÉ DE FABRICATION DE MICROSTRUCTURES

(30) Priorität: 22.08.2019 DE 102019122648
(43) Veröffentlichungstag der Anmeldung: 29.06.2022
(73) Patentinhaber: LTS Lohmann Therapie-Systeme AG, 56626 Andernach (DE)
(72) Erfinder: KULIK, Michael, 56220 Urmitz (DE); TISSIN, Nikolaj, 52249 Eschweiler (DE)
(74) Vertreter: dompatent von Kreisler Selting Werner - Partnerschaft von Patent- und Rechtsanwälten mbB
(86) Internationale Anmeldenummer: PCT/EP2020/073039
(87) Internationale Veröffentlichungsnummer: WO 2021/032701

(56) Entgegenhaltungen:
- EP-A1- 2 090 331
- WO-A1-2005/082596
- WO-A1-2020/148068
- WO-A2-2006/062974
- KR-B1- 101 697 556
- US-A1- 2002 082 543
- US-A1- 2011 152 792
- US-A1- 2013 144 217
- US-A1- 2017 050 010

## Beschreibung

Die Erfindung betrifft eine Vorrichtung sowie ein Verfahren zur Herstellung von Mikrostrukturen, insbesondere Mikronadeln.

Bei den herzustellenden Mikrostrukturen handelt es sich vorzugsweise um Mikronadeln, die insbesondere in ein Mikronadel-Array angeordnet sind. Mikronadeln werden eingesetzt, um Wirkstoffe direkt in die Haut, auch als transdermale Verabreichung bezeichnet, abzugeben. Hierzu weisen die Mikronadeln gerade eine derartige Länge auf, um lediglich in die äußeren Hautschichten einzudringen, jedoch vorzugweise Nerven und Blutgefäße nicht zu erreichen und somit diese unverletzt zu belassen. Nichtsdestotrotz erzeugen Mikronadeln kleine Löcher in den oberen Hautschichten, wodurch die Wirkstoffaufnahme gegenüber einer rein äußerlichen Auftragung von Wirkstoffen auf die Haut signifikant erhöht ist.

Mikronadel-Arrays, die eine Vielzahl von Mikronadeln, beispielsweise angebracht an einer Trägerfläche, aufweisen, können zur kurzfristigen Verabreichung oder zur langfristigen Applikation verwendet werden. Eine bevorzugte Möglichkeit der Wirkstoffabgabe, von den Mikronadeln in die Haut besteht darin, dass sich wirkstoffaufweisende Bereiche der Mikronadeln oder die gesamte Mikronadel auf- bzw. ablösen und derart über die Haut vom Körper aufgenommen werden. Hierzu sind die Mikronadeln insbesondere, zumindest teilweise, aus wasserlöslichen Stoffen bzw. Materialien hergestellt. Neben der direkten Wirkstoffabgabe durch die Mikronadeln an sich ist es auch möglich, dass die Mikronadeln Poren oder Hohlräume aufweisen oder als Hohlnadeln ausgebildet sind, um derart eine Wirkstoffabgabe an die Haut zu ermöglichen. Darüber hinaus können Mikronadeln an sich auch wirkstofffrei sein. Hierbei kann dann beispielsweise ein äußeres Auftragen des Wirkstoffs auf die Außenseite der Mikronadeln erfolgen oder erst nach dem Entfernen der Mikronadeln von der Haut eine wirkstoffhaltige Substanz auf die entsprechende Hautstelle aufgetragen werden, um derart Wirkstoffe mittels Einsatz von Mikronadeln zu verabreichen.

Mikronadeln können unter anderem aus Keramik, Metall oder Polymer hergestellt sein. Bevorzugt ist es, dass diesen Materialien eine oder mehrere Wirkstoffkomponenten zugegeben werden und sich derart eine Formulierung der Mikronadeln ergibt.

Bisher bekannte Verfahren zur Herstellung von therapeutischen oder diagnostischen Mikronadeln bzw. Mikronadel-Arrays eignen sich nicht oder nur eingeschränkt zur Herstellung in ausreichender Qualität und/oder Stückzahl.

Ein gängiges Verfahren zur Herstellung von Mikronadeln besteht in dem Gießen der Mikronadeln bzw. gesamter Mikronadel-Arrays, beispielsweise mittels Gussform aus Silikon. Insbesondere aufgrund der hydrophoben Eigenschaften zwischen der Gussform und der darauf aufgegebenen, meist flüssigen, Formulierung ergeben sich zahlreiche Probleme bei derartigen Herstellungsverfahren. Insbesondere besteht die Problematik, dass die einzuhaltenden Toleranzen der Mikronadeln äußert gering sind. Entsprechend geringe Toleranzen müssen einerseits bei den Matrizen, das heißt insbesondere Gussformen aus Silikon sichergestellt werden, andererseits müssen auch die Herstellungsprozesse mit entsprechend geringen Toleranzen erfolgen.

Die Herstellung von Mikronadeln bedingt eine aseptische Prozessierung, da der Wirkstoff offen im Produktionsumfeld verarbeitet werden muss. Eine nachträgliche Sterilisation ist bei vielen Wirkstoffen nicht möglich. Hierdurch besteht die Forderung nach einer aseptischen Herstellung, bei der sterile Einwegprodukte verwendet werden. Durch die Verwendung steriler Einwegprodukte wird das Risiko von Verunreinigungen verhindert.

Aus EP 2 090 331 ist eine Vorrichtung sowie ein Verfahren zur Herstellung eines Mikronadel-Arrays bekannt. Hierzu weist eine Form nadelförmige Vertiefungen auf. Zur Herstellung des Mikronadel-Arrays wird Material in die Vertiefungen eingepresst. Anschließend wird die Rückseite mit einer Schicht PMMA verpresst. Auf dessen Oberseite, die von den Nadeln abgewandt ist, wird der Wirkstoff aufgebracht, der in Richtung der Nadeln diffundiert.

Aufgabe der Erfindung ist es, eine Vorrichtung sowie ein Verfahren zur Herstellung von Mikrostrukturen, insbesondere Mikronadeln zu schaffen, mit den Mikrostrukturen mit sehr geringer Toleranzen hergestellt werden können.

Die Lösung der Aufgabe erfolgt erfindungsgemäß durch eine Vorrichtung gemäß Anspruch 1 bzw. ein Verfahren gemäß Anspruch 11.

Die erfindungsgemäße Vorrichtung dient insbesondere zur Herstellung von insbesondere sich auflösenden Mikrostrukturen wie Mikronadeln und besonders bevorzugt zur Herstellung von Mikronadel-Arrays. Die Vorrichtung weist eine Matrize auf, die an einer Oberseite mindestens eine, vorzugsweise kegelförmige Vertiefung aufweist. Die Vertiefung dient zur Herstellung der Mikrostruktur, insbesondere im Gussverfahren, das heißt, indem eine meist flüssige Formulierung auf die Oberseite der Matrize, insbesondere dosiert und verteilt wird und diese zur Herstellung der Mikrostruktur aushärtet.

Ferner weist die Vorrichtung einen Hohlzylinder auf. Dieser ist vorzugsweise Teil eines Basiselements oder bildet ein Basiselement aus. Erfindungsgemäß weist die Matrize ein Halteelement auf bzw. ist mit einem Halteelement verbunden, so dass die Matrize an dem Hohlzylinder befestigt bzw. gehalten werden kann.

Die Matrize überspannt bzw. überdeckt eine Öffnung des Hohlzylinders. Insbesondere liegt die Matrize in einem Ausgangszustand bzw. in einem Ruhezustand vor dem Verwenden bzw. Befüllen der Matrize mit einer Formulierung an dem Rand der Öffnung des Hohlzylinders an. Bei dem Hohlzylinder handelt es sich insbesondere um einen Zylinder mit kreisförmigem Querschnitt.

In dem Hohlzylinder ist ein Stößel angeordnet. Dieser ist in Längsrichtung des Hohlzylinders verschiebbar. Insbesondere kann der Stößel ausgehend von einer Ausgangs- oder Ruheposition in eine eingeschobene oder obere Position in den Hohlzylinder eingeschoben werden, so dass eine Oberseite des Stößels an einer Unterseite der Matrize anlegt, wobei die Unterseite der Matrize in Richtung des Inneren des Hohlzylinders weist. Besonders bevorzugt ist es hierbei, dass der Stößel soweit in den Hohlzylinder eingeschoben wird, dass die Matrize den Rand der Öffnung des Hohlzylinders nicht mehr berührt, sondern von diesem abgehoben wird. Hierdurch sind geringen Toleranzen erzielbar. Unabhängig davon, ob die Matrize in der Ausgangs- oder Ruheposition an der Öffnung des Hohlzylinders anliegt, weist die Erfindung den wesentlichen Vorteil auf, dass durch Einschieben des Stößels in den Hohlzylinder derart, dass die Matrize nur noch an der Oberseite des Stößels anliegt, die sich ggf. addierenden Toleranzen reduziert werden können. In der eingeschobenen oder oberen Position ist nur noch die Toleranz der Dicke der Matrize relevant. Toleranzen, die durch den Hohlzylinder hervorgerufen würden, sind hierdurch ausgeschlossen. Dies erlaubt eine mindere Toleranzqualität der Hohlzylinder sowie deren Halteelement, da die Prozesstoleranz über die Stößel hergestellt wird. Durch das Anheben der Matrize wird somit die Toleranzkette unterbrochen und die Toleranz auf die Dicke der Matrize reduziert.

In besonders bevorzugter Ausführungsform weist das mit der Matrize verbundene Haltelement ein längbares, insbesondere elastisch ausgebildetes Zwischenelement auf. Hierdurch ist es möglich, beim Einschieben des Stößels auf einfache Weise die Matrize von dem Rand der Öffnung des Hohlzylinders abzuheben. Wenn der Stößel zurückgezogen wird, verkürzt sich das Zwischenelement automatisch wieder, so dass die Matrize wieder am Rand der Öffnung des Hohlzylinders anliegt. In dieser Position kann die in die Vertiefung der Matrize eingebrachte Formulierung aushärten.

Des Weiteren ist es bevorzugt, dass das Halteelement ein Verbindungselement zum Verbinden mit dem Hohlzylinder aufweist. Die Verbindung kann hierbei reibschlüssig erfolgen, wobei eine formschlüssige Verbindung bevorzugt ist.

Hierzu ist das Verbindungselement vorzugsweise als Rastelement ausgebildet. Das Halteelement weist als Verbindungselement beispielsweise Ansätze auf, die in Ausnehmungen eingreifen, die an dem Hohlzylinder vorgesehen sind. Ebenso kann der Hohlzylinder Ansätze aufweisen, die in entsprechenden Ausnehmungen des Halteelements eingreifen. Hierdurch ist ein lagegenaues Anordnen der Matrize an dem Hohlzylinder möglich. In besonders bevorzugter Ausführungsform werden mit den Hohlzylindern verbundene Matrizen als sterile Einheit verwendet bzw. als sterile Einheit am Produktionsort angeliefert. Nach Herstellung der Mikrostruktur erfolgt vorzugsweise ein Entsorgen dieser Einheit ohne Stößel oder ggf. ein Sterilisieren vor einer erneuten Verwendung.

In besonders bevorzugter Ausführungsform ist das Zwischenelement mit dem Verbindungselement und mit der Matrize verbunden. Besonders bevorzugt ist es, dass die Matrize und das Zwischenelement und insbesondere die Matrize des Zwischenelements und das Verbindungselements einstückig ausgebildet sind. Als Material ist insbesondere Silikon geeignet. Besonders bevorzugt ist es, dass das Halteelement den Hohlzylinder vorzugsweise in Umfangsrichtung, insbesondere vollständig umgibt. Insbesondere bei einer einstückigen Ausgestaltung der Matrize mit dem Halteelement, gegebenenfalls zusammen mit dem Zwischenelement und dem Verbindungselement ist diese kappenförmig ausgebildet und kann beispielsweise über den Hohlzylinder gestülpt oder in den Hohlzylinder eingesteckt werden.

In besonders bevorzugter Ausgestaltung umgibt das Halteelement den Hohlzylinder an seiner Außenseite in Umfangsrichtung, insbesondere vollständig oder liegt an einer Innenseite des Hohlzylinders vorzugsweise in Umfangsrichtung vollständig an.

Bei einer besonders bevorzugten Ausführungsform der Erfindung sind mehrere Hohlzylinder vorgesehen, beispielsweise mit einem gemeinsamen Basiselement verbunden. Die Hohlzylinder sind hier insbesondere in Spalten und Reihen angeordnet und beispielsweise einstückig als Kunststoff-Spritzgusselement hergestellt. Mit jedem einzelnen Hohlzylinder ist eine Matrize verbunden, die wie vorstehend erläutert, insbesondere als Silikonkappe ausgebildet sein kann, die über einen Hohlzylinder gestülpt und/oder in einen Hohlzylinder eingesteckt wird. Des Weiteren ist es bevorzugt, dass mehrere Stößel vorgesehen sind, die insbesondere als Gruppe ebenfalls miteinander verbunden und gemeinsam verschoben werden.

Hierbei kann die Anzahl der Stößel der Anzahl der Hohlzylinder entsprechen, so dass beim Einschieben sämtliche Stößel in die Hohlzylinder sämtliche Matrizen an der Oberseite der Stößel anliegen und insbesondere vom Rand der einzelnen Hohlzylinder abgehoben werden. Die Gruppe an Stößel kann jedoch auch eine geringere Anzahl als die Anzahl der Hohlzylinder aufweisen, so dass stets eine gruppenweise Herstellung von Mikronadeln erfolgt. Beispielsweise kann eine Gruppe von vier Stößel in die Hohlzylinder eingeschoben werden, so dass die Matrizen entsprechend an der Oberseite des Stößels anliegen und insbesondere vom Rand des Hohlzylinders abgehoben werden. Anschließend erfolgt ein Befüllen der Vertiefungen in den Matrizen durch eine insbesondere von Düsen aufgebrachte Formulierung. Im nächsten Schritt werden die Stößel zurückgezogen, so dass die Matrizen wieder an den Rändern der Hohlzylinder anliegen. Anschließend erfolgt ein Verfahren der Gruppe Stößel und/oder der Gruppe an Hohlzylindern, so dass die Stößel unter einer weiteren Gruppe an Matrizen angeordnet sind und das entsprechende Verfahren wiederholt werden kann.

Die Erfindung betrifft ferner ein Verfahren zur Herstellung von Mikrostrukturen, insbesondere Mikronadeln und besonders bevorzugt Mikronadel-Arrays. Das Verfahren wird vorzugsweise mit der vorstehend beschriebenen Vorrichtung durchgeführt.

In einem ersten Schritt erfolgt ein Einschieben des mindestens einen Stößels in den jeweils zugehörigen Hohlzylinder, so dass eine Oberseite des Stößels an einer Unterseite der Matrize anliegt. Abschließend erfolgt ein Befüllen der mindestens einen Vertiefung der Matrize mit einer Formulierung. Als Formulierung ist insbesondere PVP, PLGA, Polymer, Zucker geeignet. Anschließend erfolgt ein Zurückziehen des mindestens einen Stößels und insbesondere nach dem Aushärten ein Entnehmen der Mikrostruktur aus der Matrize.

Der mindestens eine Stößel wird vorzugsweise derart in den Hohlzylinder eingeschoben, dass die Matrize von dem die Öffnung des Hohlzylinders umgebenden Rand abgehoben wird, um die erzielbaren geringen Toleranzen zu erreichen.

Vorzugsweise verkürzt sich beim Zurückziehen des mindestens einen Stößels das Zwischenelement des mit der Matrize verbundenen insbesondere einstückig ausgebildeten Zwischenelements des Halteelements derart, dass die Unterseite der Matrize wieder auf dem die Öffnung des Hohlzylinders umgebenden Rand aufliegt. In dieser Position kann die in die mindestens eine Vertiefung eingebrachte Formulierung aushärten.

Bevorzugt ist es, dass mehrere Matrizen vorhanden sind, die einzeln nacheinander oder in Gruppen befüllt werden, wobei das erfindungsgemäße Verfahren wie vorstehend anhand der Vorrichtung beschrieben vorteilhaft weitergebildet ist.

Nachfolgend wird die Erfindung anhand einer bevorzugten Ausführungsform unter Bezugnahme auf die anliegenden Zeichnungen näher erläutert.

Es zeigen:
- Fig. 1: eine schematische Schnittansicht einer ersten bevorzugten Ausführungsform der Vorrichtung,
- Fig. 2: eine schematische Schnittansicht einer zweiten bevorzugten Ausführungsform der Vorrichtung, und
- Fig. 3 und 4: schematische perspektivische Ansichten einer weiteren erfindungsgemäßen Ausführungsform der Vorrichtung, bei der eine Vielzahl von Einzelvorrichtungen wie in den Fig. 1 oder 2 dargestellt miteinander kombiniert ist.

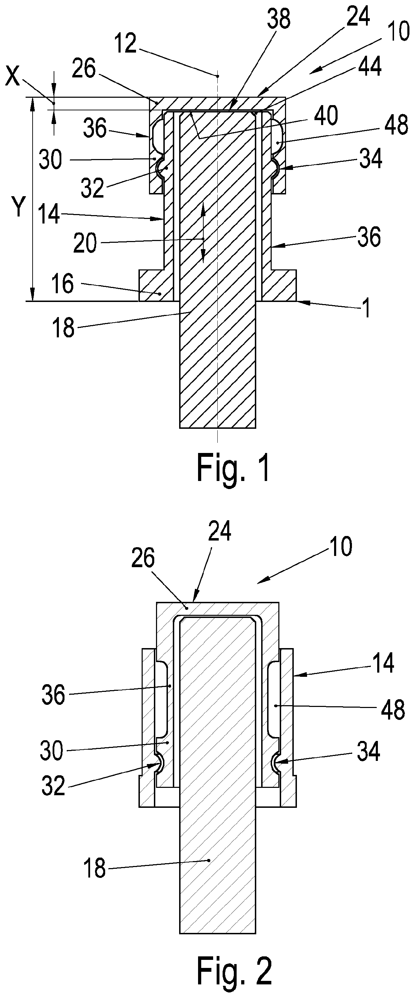

Die in Fig. 1 schematisch dargestellte erste bevorzugte Ausführungsform der Erfindung weist eine insbesondere aus einem Silikonmaterial hergestellte Kappe 10 auf, die zu einer Mittellinie 12 rotationssymmetrisch ausgebildet ist. Die Kappe 10 ist auf einem Hohlzylinder 14 angeordnet, der mit einem Basiselement 16 verbunden ist. Der Hohlzylinder 14 ist im dargestellten Ausführungsbeispiel kreiszylindrisch ausgebildet und zur Längsachse 12 rotationssymmetrisch.

Innerhalb des Hohlzylinders 14 ist ein Stößel 18 in Richtungen des Pfeils 20 verschiebbar angeordnet. Der Stößel 18 ist kreiszylindrisch und ebenfalls zur Längsachse rotationssymmetrisch.

Die Kappe 10 ist im dargestellten Ausführungsbeispiel einstückig ausgebildet und weist eine Matrize 22 auf, an deren Oberseite 24 insbesondere eine Vielzahl von vorzugsweise kegelstumpfförmigen Vertiefungen vorgesehen ist. Hierdurch ist durch Befüllen mit einer Formulierung die Herstellung eines Mikronadel-Arrays möglich. Die Matrize 22 ist im dargestellten Ausführungsbeispiel von einem ringförmigen, den Rand der Matrize versteifenden Versteifungselement 26 umgeben. Dies ist mit einem kreisförmig ausgebildeten Zwischenelement 28 verbunden, durch das eine Verbindung zu einem Halteelement 30 erfolgt. Das Halteelement 30 wird im dargestellten Ausführungsbeispiel durch formschlüssige Verbindungselemente 32, 34 mit einer Außenseite 36 des Hohlzylinders 14 verbunden. Die Verbindungselemente sind als beispielsweise ringförmige Wulst 32 ausgebildet, die an der Außenseite des Hohlzylinders 14 vorgesehen ist und mit einer entsprechenden ringförmigen Ausnehmung 34 entsprechend einer Rastverbindung zusammenwirkt.

Da die gesamte Kappe 10 im dargestellten Ausführungsbeispiel aus einem Silikonmaterial hergestellt ist und das Zwischenelement 28 eine geringere Wandstärke aufweist, handelt es sich bei dem Zwischenelement 28 um ein längbares, insbesondere elastisch ausgebildetes Zwischenelement. Zwischen dem Zwischenelement 28 und dem Hohlzylinder 14 ist ein Spalt 48 ausgebildet.

Durch Verschieben des Stößels 18 in Fig. 1 nach oben, gelangt eine plane Oberseite 38 des Stößels in Anlage mit einer Innenseite 40 der Matrize 22. Hierdurch wird die Matrize 22 von einem Rand 42 des Hohlzylinders, der eine Öffnung 44 des Hohlzylinders umgibt, abgehoben.

Bei einer alternativen Ausführungsform liegt die Innenseite der Matrize 22 nicht an dem Rand 42 des Hohlzylinders an. Dennoch erfolgt durch das Anheben der Matrize 22 durch den Stößel 18 ein unterbrechen der Toleranzkette, sodass nur noch die Toleranz der Dicke der Matrize 22 relevant ist und die Toleranzen des Hohlzylinders 14 nicht mehr berücksichtigt werden müssen, bzw. die Qualität der herzustellenden Mikrostruktur nicht negativ beeinflussen.

In diesem abgehobenen Zustand der Matrize 22 kann auf die Oberseite der Matrize eine Formulierung, insbesondere durch Aufgespritzt aufgebracht werden, so dass das aufgespritzte Material in die Vertiefungen eindringt. Anschließend erfolgt ein Zurückziehen des Stößels 18, so dass die Innenseite 40 der Matrize 22 wieder am Rand 42 des Hohlzylinders 14 anliegt und in dieser Position aushärten kann.

Bei der in Fig. 2 dargestellten alternativen Ausführungsform sind ähnliche oder identische Bauteile mit denselben Bezugszeichen gekennzeichnet.

Der wesentliche Unterschied der beiden in den Fign. 1 und 2 dargestellten Ausführungsformen besteht darin, dass die Silikonkappe 10 der in Fig. 1 dargestellten Ausführungsform den Hohlzylinder 14 umgibt bzw. auf diesen aufgestülpt werden kann und in Fig. 2 innerhalb des Hohlzylinders 14 angeordnet ist. Insofern ist insbesondere das Halteelement 30 der Kappe sowie die Verbindungselemente 32, 34 innerhalb des Hohlzylinders 14 angeordnet. Bei dieser Ausführungsform liegt die Matrize 22 nicht an einem Rand des Hohlzylinders 14 an. Im Übrigen ist die Funktion identisch.

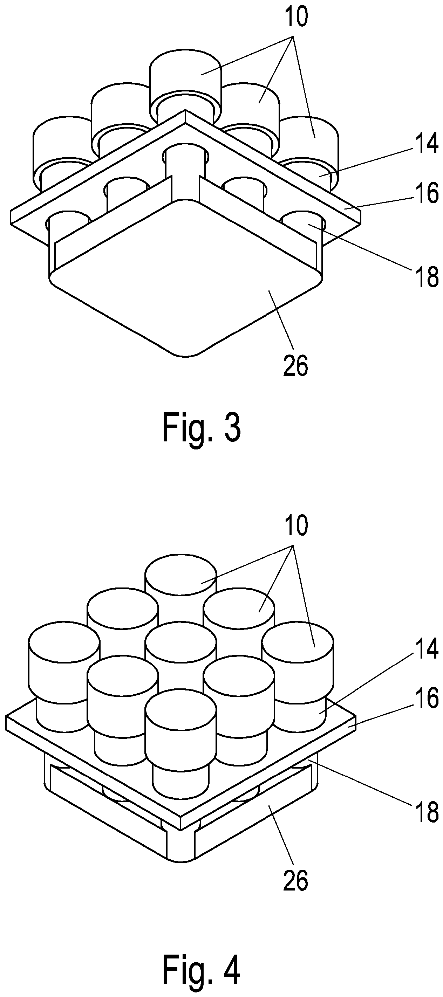

In Fig. 3 und 4 sind perspektivische schematische Ansichten eines Feldes bzw. Arrays von einer Vielzahl von Vorrichtungen dargestellt, wie sie in Fig. 1 oder 2 sichtbar sind. Hierbei sind eine Vielzahl von Hohlzylindern 14 über das Basiselement 16 miteinander verbunden. Eine im dargestellten Ausführungsbeispiel entsprechende Anzahl an Stößel 18 ist über ein insbesondere plattenförmiges Verbindungelement 26 miteinander verbunden. An Stelle der schematisch in den Figuren 3 und 4 dargestellten Feldes mit 3x3 Hohlzylindern und Stößeln kann auch ein deutlich größeres Feld von beispielsweise 10x10, 15x15, 20x20 Hohlzylindern und Stößeln vorgesehen sein.

Anstelle des Vorsehens eines Feldes mit einer entsprechenden Anzahl an Stößel 18 kann auch eine Gruppe von beispielsweise 4, 8 oder 16 Stößel vorgesehen sein, so dass jeweils ein Teil der Matrizen durch die Stößel angehoben, mit Material befüllt und anschließend die Stößel wieder zurückgezogen werden. Anschließend erfolgt ein Verfahren des Matrizenfeldes, und/oder der Gruppe an Stößeln.

## Patentansprüche

1. Vorrichtung zur Herstellung von Mikrostrukturen, insbesondere Mikronadeln, mit
einer Matrize (22), die an einer Oberseite (24) mindestens eine, vorzugsweise kegelförmige Vertiefung zur Herstellung einer Mikrostruktur aufweist,
einem, einen Hohlzylinder (14) aufweisenden Basiselement (16),
einem mit der Matrize (22) verbundenen Halteelement (30) zum Halten der Matrize (22) an dem Hohlzylinder (14) derart, dass die Matrize (22) eine Öffnung (44) des Hohlzylinders (14) überspannt und
einem in dem Hohlzylinders (14) angeordneten in Längsrichtung (20) des Hohlzylinders (14) derart verschiebbaren Stößel (18), dass eine Oberseite (38) des Stößels (18) an einer Unterseite (40) der Matrize (22) anliegt.

2. Vorrichtung nach Anspruch 1, **dadurch gekennzeichnet, dass** das Haltelement (30) ein längbares, insbesondere elastisches Zwischenelement (28) aufweist bzw. mit einem Zwischenelement (28) verbunden ist.

3. Vorrichtung nach einen der Ansprüche 1 oder 2, **dadurch gekennzeichnet, dass** das Halteelement (30) ein Verbindungselement (32, 34) zum insbesondere formschlüssigen Verbinden mit dem Hohlzylinder (14) aufweist bzw. mit diesem verbunden ist.

4. Vorrichtung nach Anspruch 3, **dadurch gekennzeichnet, dass** das Zwischenelement (28) mit dem Verbindungselement (32, 34) und der Matrize (22) verbunden ist, insbesondere einstückig ausgebildet ist.

5. Vorrichtung nach einen der Ansprüche 1 - 4, **dadurch gekennzeichnet, dass** das Haltelement (32) vorzugsweise in Umfangsrichtung vollständig den Hohlzylinder (14) umgibt.

6. Vorrichtung nach einen der Ansprüche 1 - 4, **dadurch gekennzeichnet, dass** das Halteelement (32, 34) an einer Innenseite des Hohlzylinders (14) vorzugsweise in Umfangsrichtung vollständig anliegt.

7. Vorrichtung nach einen der Ansprüche 3 - 5, **dadurch gekennzeichnet, dass** zwischen dem Verbindungselement (28) und dem Hohlzylinder (14) ein Spalt (48) ausgebildet ist.

8. Vorrichtung nach einen der Ansprüche 1 - 7, **dadurch gekennzeichnet, dass** der Stößel (18) eine ebene Oberseite (38) aufweist, die in einer eingeschobenen Position an einer Unterseite (40) der Matrize (22) anliegt.

9. Vorrichtung nach einen der Ansprüche 1 - 8, **dadurch gekennzeichnet, dass** das Basiselement (16) mehrere Hohlzylinder (14) aufweist, die vorzugsweise miteinander verbunden sind.

10. Vorrichtung nach einen der Ansprüche 1 - 9, **dadurch gekennzeichnet, dass** mehrere Stößel (18) vorgesehen sind, die vorzugsweise insbesondere über eine Verbindungsplatte (46) miteinander verbunden sind.

11. Verfahren zum Herstellen von Mikrostrukturen, insbesondere Mikronadeln, mit einer Vorrichtung nach einem der Ansprüche 1 - 10, mit den Schritten:
Einschieben des mindestens einen Stößels (18) in jeweils mindestens einen Hohlzylinder (14), so dass eine Oberseite (38) des mindestens einen Stößels (18) an einer Unterseite (40) der Matrize (22) anliegen, Befüllen der mindestens einen Vertiefung der Matrize (22) mit einer Formulierung,
Zurückziehen des Stößels (38) und
Entnehmen einer Mikrostruktur, vorzugsweise nach deren Aushärten.

12. Verfahren nach Anspruch 11, bei welchen der mindestens eine Stößel (18) derart eingeschoben wird, dass sich das Zwischenelement (28) längt und die Matrize (22) vorzugsweise von einem die Öffnung (44) des Hohlzylinders (14) umgebenden Rand (42) abgehoben wird.

13. Verfahren nach Anspruch 11 oder 12, bei welchem sich beim Zurückziehen des mindestens einen Stößels (18) das Zwischenelement (28) kürzt bzw. zusammenzieht, so dass die Unterseite (40) der Matrize (22) auf dem die Öffnung (44) umgebenden Rand (42) des Hohlzylinders (14) anliegt.

14. Verfahren nach Anspruch 11 - 13, bei welchem bei Vorhandensein mehrerer Matrizen (22) diese einzeln nacheinander, alle gemeinsam oder in Gruppen mit dem mindestens einen Stößel (38) insbesondere abgehoben werden und anschließend befüllt werden.

15. Verfahren nach einen der Ansprüche 11 - 14, dass die mindestens eine Matrize (22) über eine Düseneinrichtung mit einer Formulierung befüllt wird.

## Claims

1. Device for producing microstructures, in particular microneedles, comprising
a female mold (22) comprising at least one preferably conical cavity at the upper side (24) for producing a microstructure,
a base element (16) comprising a hollow cylinder (14),
a holding element (30), which is connected to the female mold (22), for holding the female mold (22) at the hollow cylinder (14) such that the female mold (22) spans an opening (44) of the hollow cylinder (14), and
a plunger (18) arranged in the hollow cylinder (14) and displaceable in the longitudinal direction (20) of the hollow cylinder (14) such that an upper side (38) of the plunger (18) rests on a lower side (40) of the female mold (22).

2. Device according to claim 1, **characterized in that** the holding element (30) comprises an elongatable, in particular elastic intermediate element (28) or is connected to an intermediate element (28).

3. Device according to one of claims 1 or 2, **characterized in that** the holding element (30) comprises or is connected to a connecting element (32, 34) for an in particular positive connection to the hollow cylinder (14).

4. Device according to claim 3, **characterized in that** the intermediate element (28) is connected to the connecting element (32, 34) and the female mold (22), in particular formed in one piece therewith.

5. Device according to one of claims 1-4, **characterized in that** the holding element (32) surrounds the hollow cylinder (14) preferably completely in the circumferential direction.

6. Device according to one of claims 1-4, **characterized in that** the holding element (32, 24) fully rests on an inner side of the hollow cylinder (14), preferably in the circumferential direction.

7. Device according to one of claims 3-5, **characterized in that** a gap (48) is formed between the connecting element (28) and the hollow cylinder (14).

8. Device according to one of claims 1-7, **characterized in that** the plunger (18) has a planar upper side (38) which, in an inserted position, rests on a lower side (40) of the female mold (22).

9. Device according to one of claims 1-8, **characterized in that** the base element (16) comprises a plurality of hollow cylinders (14) which cylinders are preferably connected to one another.

10. Device according to one of claims 1-9, **characterized in that** a plurality of plungers (18) is provided, which plungers are preferably connected to one another via a connecting plate (46).

11. Method for producing microstructures, in particular microneedles, with a device according to one of claims 1 - 10, the method comprising the steps of:
inserting the at least one plunger (18) into at least one hollow cylinder (14) respectively, so that an upper side (38) of the at least one plunger (18) rests on a lower side (40) of the female mold (22),
filling the at least one cavity of the female mold (22) with a formulation,
retracting the plunger (18), and
removing a microstructure, preferably after the curing thereof.

12. Method according to claim 11, wherein the at least one plunger (18) is inserted such that the intermediate element (28) is elongated and the female mold (22) is preferably lifted off from an edge (42) surrounding the opening (44) of the hollow cylinder (14).

13. Method according to claim 11 or 12, wherein, when the at least one plunger (18) is retracted, the intermediate element (28) shortens or contracts, so that the lower side (40) of the female mold (22) rests on the edge (42) of the hollow cylinder (14) surrounding the opening (44).

14. Method according to one of claims 11-13, wherein, if a plurality of female molds (22) is present, these are, either individually one after the other, all together or in groups, in particular lifted off by the at least one plunger (18) and filled subsequently.

15. Method according to one of claims 11-14, wherein the at least one female mold (22) is filled with a formulation via a dosing means.

## Revendications

1. Dispositif de production de microstructures, en particulier de micro-aiguilles, avec
une matrice (22) comprenant sur une face supérieure (24) au moins un renfoncement, de manière préférée conique, permettant de produire une microstructure,
un élément de base (16) comprenant un cylindre creux (14),
un élément de retenue (30) relié à la matrice (22) et permettant de retenir la matrice (22) sur le cylindre creux (14) de telle manière que la matrice (22) recouvre une ouverture (44) du cylindre creux (14) et
un piston (18) agencé dans le cylindre creux (14) et pouvant être déplacé dans la direction longitudinale (20) du cylindre creux (14) de telle manière qu'une face supérieure (38) du piston (18) repose sur une face inférieure (40) de la matrice (22).

2. Dispositif selon la revendication 1, **caractérisé en ce que** l'élément de retenue (30) comprend un élément intermédiaire (28) extensible, en particulier élastique, ou est relié à un élément intermédiaire (28).

3. Dispositif selon l'une quelconque des revendications 1 ou 2, **caractérisé en ce que** l'élément de retenue (30) comprend un élément de liaison (32, 34) permettant une liaison, en particulier par complémentarité de forme, avec le cylindre creux (14) ou est relié audit élément de liaison.

4. Dispositif selon la revendication 3, **caractérisé en ce que** l'élément intermédiaire (28) est relié à l'élément de liaison (32, 34) et à la matrice (22), en particulier est réalisé d'un seul tenant.

5. Dispositif selon l'une quelconque des revendications 1 à 4, **caractérisé en ce que** l'élément de retenue (32) entoure de manière préférée complètement le cylindre creux (14) dans le sens circonférentiel.

6. Dispositif selon l'une quelconque des revendications 1 à 4, **caractérisé en ce que** l'élément de retenue (32, 34) repose complètement sur une face intérieure du cylindre creux (14), de manière préférée dans la direction circonférentielle.

7. Dispositif selon l'une quelconque des revendications 3 à 5, **caractérisé en ce qu'**une fente (48) est formée entre l'élément de liaison (28) et le cylindre creux (14).

8. Dispositif selon l'une quelconque des revendications 1 à 7, **caractérisé en ce que** le piston (18) comprend une face supérieure (38) plane qui, dans une position insérée, repose sur une face inférieure (40) de la matrice (22).

9. Dispositif selon l'une quelconque des revendications 1 à 8, **caractérisé en ce que** l'élément de base (16) comprend plusieurs cylindres creux (14) qui sont de manière préférée reliés les uns aux autres.

10. Dispositif selon l'une quelconque des revendications 1 à 9, **caractérisé en ce que** plusieurs pistons (18) sont fournis, qui sont reliés l'un à l'autre de manière préférée en particulier par l'intermédiaire d'une plaque de liaison (46).

11. Procédé de fabrication de microstructures, en particulier de micro-aiguilles, avec un dispositif selon l'une quelconque des revendications 1 à 10, comprenant les étapes consistant à :
insérer ledit au moins un piston (18) dans respectivement au moins un cylindre creux (14), de sorte qu'une face supérieure (38) dudit au moins un piston (18) repose sur une face inférieure (40) de la matrice (22),
remplir ledit au moins un renfoncement de la matrice (22) avec une formulation,
reculer le piston (38) et
retirer une microstructure, de manière préférée après son durcissement.

12. Procédé selon la revendication 11, dans lequel ledit au moins un piston (18) est inséré de telle manière que l'élément intermédiaire (28) s'allonge et que la matrice (22) est soulevée de manière préférée par un bord (42) entourant l'ouverture (44) du cylindre creux (14).

13. Procédé selon la revendication 11 ou 12, dans lequel l'élément intermédiaire (28) se raccourcit ou recule lors du reculement dudit au moins un piston (18), de sorte que la face inférieure (40) de la matrice (22) repose sur le bord (42), entourant l'ouverture (44), du cylindre creux (14).

14. Procédé selon les revendications 11 à 13, dans lequel, lorsque plusieurs matrices (22) sont comprises, lesdites matrices sont en particulier soulevées, individuellement les unes après les autres, toutes ensemble ou par groupes avec ledit au moins un piston (38), et ensuite remplies.

15. Procédé selon l'une quelconque des revendications 11 à 14, dans lequel ladite au moins une matrice (22) est remplie d'une formulation par l'intermédiaire d'un dispositif de buse.
